# EUROPÄISCHE PATENTSCHRIFT

(11) **EP 2 828 235 B1**
(45) Veröffentlichungstag und Bekanntmachung des Hinweises auf die Patenterteilung: **22.11.2017**
(21) Anmeldenummer: 13709242.5
(22) Anmeldetag: 15.03.2013
(51) Int. Cl.: C07C 263/10, C07C 263/20

(54) **VERFAHREN ZUR HERSTELLUNG VON ISOCYANATEN**
METHOD FOR MAKING ISOCYANATES
PROCÉDÉ DESTINÉ À LA FABRICATION D'ISOCYANATES

(30) Priorität: 19.03.2012 EP 12160168
(43) Veröffentlichungstag der Anmeldung: 28.01.2015
(73) Patentinhaber: Covestro Deutschland AG, 51373 Leverkusen (DE)
(72) Erfinder: BRUNS, Rainer, 51373 Leverkusen (DE); LORENZ, Wolfgang, 41540 Dormagen (DE); RAUSCH, Andreas Karl, 41564 Kaarst (DE); WERSHOFEN, Stefan, 41065 Mönchengladbach (DE); LODDENKEMPER, Tim, 41542 Dormagen (DE)
(74) Vertreter: Levpat
(86) Internationale Anmeldenummer: PCT/EP2013/055411
(87) Internationale Veröffentlichungsnummer: WO 2013/139703

(56) Entgegenhaltungen:
- WO-A1-2009/027418
- DE-A1- 2 844 591

## Beschreibung

Gegenstand der Erfindung ist ein Verfahren zur Herstellung von Isocyanaten durch Phosgenierung der korrespondierenden Amine, worin die Abtrennung von leichtsiedenden Nebenkomponenten, überschüssigem Phosgen und des Koppelproduktes Chlorwasserstoff aus dem nach erfolgter Phosgenierung erhaltenen flüssigen Roh-Isocyanatstrom innerhalb von maximal 60 Minuten erfolgt, und worin der flüssige Roh-Isocyanatstrom bis zu dieser Abtrennung keinen Temperaturen von über 250 °C ausgesetzt wird.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen, wobei neben dem Isocyanat auch Chlorwasserstoff gebildet wird.

Eine weit verbreitete Möglichkeit der Herstellung von Isocyanaten ist die Umsetzung der entsprechenden Amine mit Phosgen in der Flüssigphase. Diese auch als Flüssigphasenphosgenierung (FPP) bezeichnete Verfahrensführung zeichnet sich dadurch aus, dass üblicherweise die Umsetzung in einem inerten Lösungsmittel erfolgt, und dass die Reaktionsbedingungen so gewählt werden, dass neben dem Lösungsmittel zumindest die Reaktionskomponenten Amin, Isocyanat und Phosgen bei den gewählten Bedingungen zumindest teilweise, vorzugsweise zum überwiegenden Teil, in der flüssigen Phase vorliegen. Der bei der Reaktion als Koppelprodukt entstehende Chlorwasserstoff liegt zum Teil gelöst in der flüssigen Phase vor und verlässt den Reaktor zum Teil gasförmig. Die Flüssigphasenphosgenierung kann bei verschiedenen Temperatur- und Druckniveaus durchgeführt werden. So ist es beispielsweise möglich, die Flüssigphasenphosgenierung bei Temperaturen von 0 °C bis 240 °C und Drücken von 1 bar bis 70 bar durchzuführen; in einigen Fällen werden Temperaturen bis zu 300°C und Drücke bis zu 300 bar beschrieben.

Eine effiziente Vermischung von Amin und Phosgen ist beim Flüssigphasenverfahren von hoher Bedeutung. Hierzu kommen im Stand der Technik statische (bevorzugt Düsen) und dynamische (enthaltend mechanisch bewegte Teile) Mischeinrichtungen zum Einsatz. Aus EP 0 291 819 A und EP 0 291 820 A und EP 0 830 894 A sind Mischer-Reaktoren bekannt, die aus einem im Wesentlichen rotationssymmetrischen Gehäuse bestehen, wobei das Gehäuse eine im Wesentlichen rotationssymmetrische Mischkammer mit getrennten Einlässen für die mindestens zwei Einsatzstoffströme und einen Auslass aufweist. Der Einlass für wenigstens einen ersten Stoffstrom ist in der Achse der Mischkammer vorgesehen und der Einlass für wenigstens einen zweiten Stoffstrom ist in Form einer Vielzahl von rotationssymmetrisch zur Mischkammerachse angeordneten Düsen ausgebildet. Zur besseren Vermischung enthalten die Mischer-Reaktoren noch mindestens eine Rotorscheiben-Statorscheiben-Einheit sowie ein Laufrad, um die Förderwirkung im Mischer-Reaktor zugunsten eines engeren Verweilzeitspektrums zu verbessern. Es ist auch möglich, auf das Laufrad der vorstehend beschriebenen Mischer-Reaktoren zu verzichten. Dadurch wird im Vergleich zur technischen Lehre der EP 0 291 819 A, EP 0 291 820 A und EP 0 830 894 A der Druck in der Mischkammer erhöht. Zur Förderung des Vorprodukt- und/oder Produktstromes wird ausschließlich der Vordruck der Eduktströme genutzt. Eine Pumpwirkung ist nicht mehr gegeben. Es ist auch möglich, einen modifizierten Vorsatzläufer einzubauen, dergestalt, dass die Förderwirkung entgegen der Hauptförderrichtung der Eduktströme bzw. des Vorproduktstromes erfolgt. Hierdurch wird ebenfalls der Druck in der Mischkammer erhöht. Der modifizierte Vorsatzläufer ist bevorzugt auf derselben Welle wie die Rotorscheiben angeordnet. Beide Maßnahmen bewirken im Vergleich zur technischen Lehre der EP 0 291 819 A, EP 0 291 820 A und EP 0 830 894 A demnach eine Druckerhöhung in der Mischkammer.

Allen Verfahrensvarianten zur Flüssigphasenphosgenierung ist gemeinsam, dass nach Beendigung der Reaktion eine Abtrennung des überschüssigen Phosgens und des gebildeten Chlorwasserstoffs von dem in dem Lösungsmittel gelösten Roh-Isocyanat erfolgt. Diese Abtrennung erfolgt im Allgemeinen dergestalt, dass ein gasförmiger Strom enthaltend im Wesentlichen das überschüssige Phosgen und Chlorwasserstoff sowie ein flüssiger Strom enthaltend unter anderem das Lösungsmittel und das gewünschte Isocyanat den Reaktor, in dem die Umsetzung stattfindet, verlässt. Weiterhin ist allen Verfahrensvarianten der Flüssigphasenphosgenierung gemeinsam, dass der gasförmige Strom in Chorwasserstoff und überschüssiges Phosgen aufgetrennt wird und Letzteres im Allgemeinen der Reaktion wieder zugeführt wird. Die Reinigung des flüssigen Stroms enthaltend das gewünschte Isocyanat und das Lösungsmittel erfolgt im Allgemeinen destillativ. Der den Reaktor verlassende flüssige Strom enthält entsprechend den herrschenden Druck- und Temperaturverhältnissen noch gelöstes Phosgen und Chlorwasserstoff. Diese werden zusammen mit Reaktionsnebenkomponenten, deren Bildung im Reaktor nicht vollständig vermieden werden kann, in der Destillation abgetrennt. Durch die Destillation wird das gewünschte Isocyanat in hoher Reinheit erhalten.

Einen Überblick über verschiedene Varianten der Reaktionsführung sowie der Aufarbeitung und Produktgewinnung geben die Anmeldungsschriften DE-A-102 60 027, DE-A-102 60 093, DE-A 103 10 888, DE-A-10 2006 022 448, US-A 2007/0299279 und die darin zitierten Quellen.

Eine andere Möglichkeit der Herstellung von Isocyanaten ist die Umsetzung der entsprechenden Amine mit Phosgen in der Gasphase. Diese üblicherweise als Gasphasenphosgenierung (GPP) bezeichnete Verfahrensführung zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Produkte und Reaktionszwischenprodukte, bei den gewählten Bedingungen gasförmig sind.

Es sind aus dem Stand der Technik verschiedene Verfahren zur Herstellung von Di- und/oder Polyisocyanaten durch Umsetzung von Di- und/oder Polyaminen mit Phosgen in der Gasphase bekannt.

GB-A-1 165 831 beschreibt ein Verfahren zur Herstellung von Isocyanaten in der Gasphase, bei dem die Umsetzung des dampfförmigen Amins mit dem Phosgen bei Temperaturen zwischen 150 °C und 300 °C in einem mit einem mechanischen Rührer ausgestatteten und über einen Heizmantel temperierbaren Rohrreaktor durchgeführt wird. Der in GB-A-1 165 831 offenbarte Reaktor ähnelt einem Dünnschichtverdampfer, dessen Rührer die in den Reaktionsraum eintretenden sowie die in dem Reaktionsraum vorhandenen Gase mischt und gleichzeitig die mit dem Heizmantel umgebenen Wände des Rohrreaktors bestreicht, um so einen Aufbau von polymeren Material an der Rohrwand zu verhindern, da ein solcher Aufbau den Wärmeübergang erschweren würde. Die Schrift offenbart nicht, wie das durch den offenbarten Reaktor erhaltene Roh-Isocyanat zum reinen Isocyanat aufgereinigt wird und wie Ausbeuteverluste verringert werden können.

EP-A-0 289 840 beschreibt die Herstellung von Diisocyanaten durch Gasphasenphosgenierung, wobei diese Schrift die Umsetzung der Amine mit dem Phosgen in einem zylindrischen Raum ohne bewegte Teile in einer turbulenten Strömung bei Temperaturen zwischen 200 °C und 600 °C sowie Reaktionszeiten in der Größenordnung von 10⁻⁴ Sekunden offenbart. Nach der Lehre von EP-A-0 289 840 werden die Gasströme an einem Ende des Rohrreaktors in diesen durch eine Düse und einen Ringspalt zwischen Düse und Mischrohr in den Reaktor eingebracht und dadurch vermischt. Gemäß der Lehre der EP-A-0 289 840 ist es für die Durchführbarkeit des dort offenbarten Verfahrens wesentlich, dass die Abmessungen des Rohrreaktors und die Strömungsgeschwindigkeiten im Reaktionsraum so bemessen werden, dass in dem Reaktionsraum eine turbulente Strömung herrscht, die durch eine Reynoldszahl von mindestens 2500, vorzugsweise mindestens 4700 charakterisiert wird. Nach der Lehre von EP-A-0 289 840 ist diese Turbulenz im Allgemeinen dann gewährleistet, wenn die gasförmigen Reaktionspartner den Reaktionsraum mit einer Strömungsgeschwindigkeit von mehr als 90 m/s durchlaufen. Das den Reaktionsraum verlassende Gasgemisch wird durch ein inertes Lösungsmittel geleitet, welches bei einer Temperatur oberhalb der Zersetzungstemperatur des dem Diamin entsprechenden Carbamidsäurechlorids gehalten wird, wobei sich das Diisocyanat dabei in dem inerten Lösemittel löst. Die Schrift beschreibt, dass das so erhaltene Roh-Diisocyanat destillativ aufgearbeitet werden kann, ohne eine Handlungsanleitung dafür zu geben. Die Schrift gibt keine Handlungsanweisung wie Ausbeuteverluste und Bildung von Nebenprodukten in dem Roh-Isocyanatstrom vermieden werden können.

EP-B-0 593 334 offenbart ein Verfahren zur Herstellung von aromatischen Diisocyanaten in der Gasphase, wobei ein Rohrreaktor verwendet wird. Darin wird eine Vermischung der Edukte durch eine Einengung der Wände erreicht. Die Umsetzung erfolgt im Temperaturbereich von 250 °C bis 500 °C. Am Austritt des Reaktors wird das Reaktionsprodukt durch Zuführung von Lösungsmittel in die flüssige Phase überführt. Die Schrift beschreibt, dass das so erhaltene Roh-Isocyanat destillativ aufgearbeitet werden kann, jedoch ohne dafür eine Handlungsanweisung zu geben. Die Schrift offenbart keine Methoden wie Ausbeuteverluste während der Aufarbeitung des Roh-Isocyanates vermieden oder verringert werden können.

EP-A-0 570 799 betrifft ein Verfahren zur Herstellung von aromatischen Diisocyanaten, dadurch gekennzeichnet, dass die Umsetzung des zugehörigen Diamins mit dem Phosgen in einem Rohrreaktor oberhalb der Siedetemperatur des Diamins innerhalb einer mittleren Kontaktzeit der Reaktanden von 0,5 bis 5 Sekunden durchgeführt wird. Wie in der Schrift beschrieben ist, führen sowohl zu lange als auch zu kurze Reaktionszeiten zu einer unerwünschten Feststoffbildung. Es wird daher ein Verfahren offenbart, bei dem die mittlere Abweichung von der mittleren Kontaktzeit weniger als 6 % beträgt. Auch in diesem offenbarten Verfahren wird durch Einsatz von Lösungsmittel am Austritt des Reaktors eine flüssige Phase enthaltend unter anderem Roh-Isocyanat und eine gasförmige Phase enthaltend Phosgen und Chlorwasserstoff erhalten. Eine Handlungsanweisung zur Reinigung des Roh-Isocyanates liefert die Schrift nicht.

Die Einhaltung der offenbarten Kontaktzeitverteilung wird zum einen dadurch erreicht, dass die Reaktion in einer Rohrströmung durchgeführt wird, die entweder durch eine Reynolds-Zahl von oberhalb 4.000 oder einer Bodenstein-Zahl von oberhalb 100 charakterisiert wird. Nach der Lehre von EP-A-0 570 799 wird dadurch eine zu 90 % angenäherte Pfropfenströmung erreicht; ferner weisen alle Volumenteile der Strömung weitgehend die gleichen Strömungszeiten auf, so dass durch die annähernd gleichen Verweilzeiten aller Volumenteile eine möglichst geringe Verbreiterung der Verteilung der Kontaktzeit zwischen den Reaktionspartnern erfolgt.

Nach der Lehre von EP-A-0 570 799 wird zum anderen die Abweichung von der mittleren Kontaktzeit bei der praktischen Durchführung des Verfahrens jedoch auch wesentlich durch die notwendige Zeit zur Vermischung der Reaktionspartner bestimmt. EP-A-0 570 799 führt aus, dass, solange die Reaktionspartner noch nicht homogen vermischt sind, im Reaktionsraum noch Gasvolumina vorhanden sind, die noch nicht mit dem Reaktionspartner in Kontakt treten konnten und somit abhängig von der Durchmischung bei gleichen Strömungszeiten der Volumenteile unterschiedliche Kontaktzeiten der Reaktionspartner erhalten werden. Nach der Lehre von EP-A-0 570 799 soll daher die Vermischung der Reaktionspartner innerhalb einer Zeit von 0,1 s bis 0,3 s bis zu einem Segregationsgrad von 10⁻³ erfolgen, wobei der Segregationsgrad als Maß für die Unvollständigkeit der Durchmischung dient (siehe z.B. Chem.-Ing.-Techn. 44 (1972), S.1051 ff; Appl.Sci.Res. (the Hague) A3 (1953), p. 279). EP-A-0 570 799 offenbart, dass zur Erzeugung entsprechend kurzer Mischzeiten im Prinzip bekannte Methoden auf Basis von Mischaggregaten mit bewegten oder statischen Mischorganen, bevorzugt statischen Mischorganen, eingesetzt werden können, wobei nach der Lehre von EP-A-0 570 799 insbesondere die Anwendung des Strahlmischerprinzips ausreichend kurze Mischzeiten liefert.

Somit wird auch bei der Gasphasenphosgenierung nach Beendigung der Reaktion ein flüssiger Strom enthaltend unter anderem neben dem zur Verflüssigung des Produktgasstromes und ggf. zum Reaktionsabbruch eingesetzten Lösungsmittel das Roh-Diisocyanat sowie ein gasförmiger Strom enthaltend im Wesentlichen überschüssiges Phosgen sowie Chlorwasserstoff erhalten. Der gasförmige Strom wird im Allgemeinen in Chlorwasserstoff und Phosgen aufgetrennt und das Phosgen zumindest teilweise wieder in der Reaktion eingesetzt. Der den Reaktor verlassende flüssige unter anderem das gewünschte Isocyanat enthaltende Strom wird im Allgemeinen destillativ aufgereinigt, um Rein-Isocyanat zu erhalten.

Der wesentliche Verfahrensunterschied zwischen der Flüssigphasenphosgenierung und der Gasphasenphosgenierung liegt somit in den Reaktionsbedingungen im Reaktor. Gemeinsam ist beiden Verfahrensvarianten zur Herstellung von Isocyanaten, dass nach Beendigung der Reaktion zunächst ein Rohprodukt, umfassend das gewünschte Isocyanat, inertes Lösungsmittel, Nebenkomponenten, Chlorwasserstoff und nicht umgesetztes Phosgen erhalten wird, welches in einen gasförmigen und einen flüssigen Produktstrom aufgespaltet wird, wobei der flüssige Produktstrom unter anderem das Lösungsmittel und das Roh-Isocyanat enthält und dieser flüssige Strom im Allgemeinen destillativ aufgearbeitet wird.

Trotz deutlicher Verbesserungen der Reaktionen sowohl der Flüssigphasen- als auch der Gasphasenphosgenierung sowohl durch verbesserte Mischtechniken und verbesserte Reaktionsführungen ist die Bildung von hoch- und leichtsiedenden Reaktionsnebenkomponenten in der Reaktion nicht vollständig zu vermeiden. Im Rahmen der vorliegenden Erfindung werden alle Stoffe oder azeotrop siedende Stoffgemische, deren Siedepunkte bei den im jeweiligen Zusammenhang herrschenden Bedingungen von Druck und Temperatur *unter* dem des gewünschten Isocyanats liegen, als "leichtsiedend" bezeichnet. Alle Stoffe oder azeotrop siedende Stoffgemische, deren Siedepunkte bei den im jeweiligen Zusammenhang herrschenden Bedingungen von Druck und Temperatur *über* dem des gewünschten Isocyanats liegen, werden als "hochsiedend" bezeichnet. Als "leichtsiedend" gelten im Sinne der vorliegenden Erfindung daher das Koppelprodukt Chlorwasserstoff und nicht umgesetztes Phosgen. Reaktion*snebenkomponenten* (d. h. die Produkte unerwünschter Nebenreaktionen) werden im Rahmen der vorliegenden Erfindung als "Leichtsieder" bezeichnet, sofern sie die oben genannte Definition von "leichtsiedend" erfüllen, und werden als "Hochsieder" bezeichnet, sofern sie die oben genannte Definition von "hochsiedend" erfüllen. Das - entweder in der Reaktion bereits eingesetzte oder erst später zugesetzte - Lösungsmittel fällt üblicherweise in die Gruppe der leichtsiedenden Stoffe (es gilt jedoch nicht als "Leichtsieder" im oben genannten Sinne, weil es keine Reaktionsnebenkomponente ist). Es ist jedoch auch denkbar, dass ein Lösungsmittel verwendet wird, das der Gruppe der hochsiedenden Stoffe zuzuordnen ist (es gilt jedoch nicht als "Hochsieder" im oben genannten Sinne, weil es keine Reaktionsnebenkomponente ist).

Neben den im Reaktor bei beiden Verfahren stattfindenden Nebenreaktionen sind auch die Nebenreaktionen zu beachten, die in dem den Reaktor verlassenden flüssigen, das Roh-Isocyanat enthaltenden Produktstrom stattfinden können. Dieser jeweils den Reaktor verlassende Strom enthält neben dem Roh-Isocyanat, Lösungsmittel, Leicht- und Hochsiedern auch Chlorwasserstoff und überschüssiges Phosgen. Insbesondere Phosgen ist ein hoch reaktives Molekül, das bis zur Abtrennung aus dem flüssigen Produktstrom mit anderen Komponenten weiter reagieren kann.

Ein grundsätzliches Problem ist, dass im Verlauf der Aufarbeitung zur Gewinnung des Rein-Isocyanats vergleichsweise große Verweilzeiten vorliegen, wodurch eine Hochsiederbildung aus dem Wertprodukt Isocyanat gefördert wird. Weiterhin können bereits in der Reaktion entstandene, hochsiedende Nebenkomponenten in die Aufarbeitung gelangen, wodurch eine weitere Hochsiederbildung aus dem Wertprodukt Isocyanat gefördert wird. Diese Nebenkomponenten haben die Eigenschaft, mit den Isocyanaten zu reagieren und somit den Anteil von Isocyanaten in einem Isocyanat / Nebenkomponentengemisch, wie es in einem Phosgenierungsreaktor anfällt, zu reduzieren. Dies führt zur Bildung von Rückständen, die mit hoch siedenden Nebenkomponenten angereichert sind. Die schwere Handhabbarkeit und die typische Zusammensetzung eines solchen Rückstands werden beispielsweise in DE-A-102 60 093 benannt.

In WO 2004/056759 A1 wird beispielsweise die zweistufige Abtrennung von Isocyanaten aus einem Isocyanat / Hochsiedergemisch (Strom 1) beschrieben. Die Ströme 2 (Sumpf) und 3 (Destillat) werden im Massenverhältnis 20 : 1 bis 1 : 1 aufgeteilt. Mit anderen Worten werden höchstens 50 % des Stromes 1 über Sumpf gezogen. Die Lösung wird aufkonzentriert, in einen Knetertrockner gepumpt und dort weiter eingedampft.

Gemäß WO 2009/027418 A1 können die genannten Ausbeuteverluste vermindert werden, wenn man die bereits im Roh-Isocyanatgemisch vorliegenden, hochsiedenden Verbindungen, wie z. B. Harnstoffe und seine durch Phosgenierung entstandenen Folgeprodukte oder durch Isocyanatfolgereaktionen gebildete Spezies, z. B. Carbodiimide, Isocyanurate, Uretdione, vor oder während der eigentlichen Destillationssequenz zur Abtrennung des Lösungsmittels und der Leichtsieder mit einem geeigneten Apparatekonzept abtrennt. Des Weiteren wurde gefunden, dass durch eine geeignete Voreindampfung Teile der hochsiedenden, das Isocyanat enthaltenden Komponenten in das Isocyanat zurückgespalten werden können, wodurch Ausbeuteverluste über den Sumpfaustrag der Destillationskolonne vermindert werden können. Weiterhin wird dadurch vermieden, dass sich monomeres Isocyanat bei der Aufarbeitung an die hochsiedenden Verbindungen anlagert, und dadurch die Ausbeute reduziert wird. Die verbleibenden Sumpfprodukte enthalten als wesentliche Bestandteile u. a. zumeist in polymerer Form vorliegende Carbodiimide und mehrkernige chlorierte Nebenkomponenten.

Aus der vorstehenden Zusammenstellung verschiedenster Patentanmeldungen wird ersichtlich, dass die dort beschriebenen Prozessvarianten entweder geeignete Apparate oder Prozessführungen bei der Phosgenierungsreaktion und der Eduktbereitstellung/-zuführung betreffen oder sich mit Varianten der Prozessführung bei der Aufarbeitung des Reaktionsgemisches einschließlich der Abtrennung von Leichtsiedern, Lösungsmittel und/oder Hochsiedern befassen. Alle beschriebenen Maßnahmen sollen dazu dienen, eine stabile Prozessführung zu ermöglichen bzw. die Bildung von die Ausbeute vermindernden Nebenkomponenten zu vermeiden oder zu reduzieren.

Trotz der vielen bereits erfolgten Arbeiten zur Optimierung der Umsetzung von Aminen mit Phosgen besteht hinsichtlich der Reduzierung von Nebenkomponenten ein weiterer Bedarf zur Verbesserung dieser Phosgenierungsreaktion. Wenig Aufmerksamkeit wurde in diesem Zusammenhang bisher auf den Reaktionsabschnitt zwischen Reaktion und der Abtrennung von leichtsiedenden Stoffen und Lösungsmittel gelegt. Überraschenderweise wurde nun gefunden, dass dieser Verfahrensabschnitt einen ausgeprägten Einfluss auf die Prozessführung hat, insbesondere hinsichtlich Sicherheit, Produktqualität und Wirtschaftlichkeit des Verfahrens.

Die erfindungsgemäße Verbesserung des Verfahrens zur Phosgenierung von Aminen erfordert das Verständnis der Zusammenhänge bei einigen der ablaufenden Nebenreaktionen, welche daher im Folgenden näher erläutert werden.

So sind zwei Reaktionswege für die Bildung von Carbodiimide wichtig. Zum einen ist die thermische Zersetzung von Isocyanaten zu nennen, bei der zwei Isocyanatgruppen unter Abspaltung von CO₂ miteinander reagieren. Diese Reaktion kann in allen Abschnitten des Prozesses ablaufen, in denen Isocyanat bei höheren Temperaturen vorliegt.

Zum anderen kann gemäß H. J. Twitchett (Chemical Society Reviews (1974), 3(2), 209-230) die Phosgenierung von als Nebenkomponenten bei der Phosgenierung entstehenden Harnstoffen nicht nur zu den entsprechenden Isocyanaten sondern ebenfalls zu Carbodiimide führen; daneben ist die Bildung verschiedener chlorhaltiger Strukturelemente möglich, z. B. Chlorformamidine, Chlorformamidin-N-carbonylchloride und/oder Isocyaniddichloride, die entlang des Reaktionsweges als Zwischenprodukte entstehen. Da durch die Verbesserung der Reaktionsführung sowohl in der Flüssig- als auch der Gasphasenphosgenierung die Bildung von Harnstoffen im Allgemeinen nur von untergeordneter Bedeutung ist, spielt dieser Weg der Carbodiimidbildung nur eine geringe Rolle. Dennoch kann eine Harnstoffbildung und die in Gegenwart von Phosgen daraus resultierende Carbodiimidbildung nicht vollständig ausgeschlossen werden.

Chlorformamidine können ebenfalls durch (reversible) Addition von Chlorwasserstoff, das z. B. im Reaktionsgemisch der Phosgenierung zugegen ist, an Carbodiimide entstehen (siehe z.B.: A.A.R. Sayigh, J. N. Tilley, H. Ulrich, Journal of Organic Chemistry (1964), 29(11), 3344 - 3347) und ihrerseits z. B. thermisch zu trisubstituierten Guanidinhydrochloriden weiterreagieren. Ähnliches gilt für Chlorformamidin-N-carbonylchloride, die durch (reversible) Addition von Phosgen, das z. B. im Reaktionsgemisch der Phosgenierung zugegen ist, an Carbodiimide entstehen (siehe z. B.: H. J. Twitchett, Chemical Society Reviews (1974), 3(2), 209-230) und thermisch wieder in Carbodiimide und Phosgen gespalten werden können.

Die dargelegten Reaktionen, nämlich thermische Carbodiimidbildung und Carbodiimidbildung durch Phosgenierung von Harnstoff sowie die Folgereaktion von Carbodiimide mit Chlorwasserstoff und / oder Phosgen zu Nebenprodukten können sowohl im Reaktor aber auch in dem den Reaktor verlassenen flüssigen, das Roh-Isocyanat enthaltenden Produktstrom stattfinden, da in diesem Strom entsprechend den am Austritt des Reaktors herrschenden Druck- und Temperaturverhältnissen stets gewisse Mengen an Chlorwasserstoff und Phosgen gelöst sind. Die Folgereaktionen mit Chlorwasserstoff und Phosgen sind auch in den Aufarbeitungs- bzw. Destillationsabschnitten möglich, solange Chlorwasserstoff und Phosgen noch nicht weitestgehend aus dem flüssigen, das Roh- Isocyanat enthaltenden Produktstrom abgetrennt sind.

Im Reaktor lässt sich trotz hoher Phosgenüberschüsse, guter Mischtechnik und der im Stand der Technik bereits diskutierten Maßnahmen die Bildung von Nebenprodukten mit Harnstoffstruktur nicht vollständig ausschließen. Da eine mögliche Folgereaktion der Harnstoffe zu Carbodiimide führt, enthält der den Reaktor verlassene flüssige, das Roh-Isocyanat enthaltende Produktstrom immer auch Carbodiimid-Anteile. Die Reaktion dieser Carbodiimide mit überschüssigem Phosgen führt somit immer zu gewissen Anteilen an Nebenkomponenten mit Chlorformamidin-N-carbonylchlorid-Strukturelementen. Diese Reaktion läuft insbesondere in der Flüssigphase in Gegenwart von gelöstem Phosgen unvermeidlich ab, solange noch in der Flüssigphase gelöstes Phosgen vorhanden ist.

Insofern ist nicht nur die in der Literatur nach dem Stand der Technik beschriebene frühzeitige Abtrennung von Hochsiedern hilfreich zur Minimierung von Ausbeuteverlusten. Auch die im flüssigen, das Roh-Isocyanat enthaltenden Produktstrom vorhandenen leichtsiedenden Stoffe, wie Leichtsieder und insbesondere das Koppelprodukt Chlorwasserstoff und nicht umgesetztes Phosgen, können zu einer Ausbeuteverringerung führen.

Die Bildung von Nebenkomponenten, unter anderem die oben genannten Carbodiimide und die chlorhaltigen Spezies, bedingt Ausbeuteverluste und damit wirtschaftliche Nachteile. Gelangen chlorhaltige Spezies in das Verfahrensprodukt, führt dies zudem zu unerwünschten, erhöhten Chlorwerten im Produkt. Darüber hinaus sind Nebenprodukte mit Chlorformamidin-N-carbonylchlorid-Strukturelementen Phosgen-Carrier, die in eigentlich Phosgen-freie Abschnitte des Verfahrens gelangen können und bei thermischer Belastung wieder Phosgen abspalten, wodurch Phosgen in eigentlich Phosgen-freie Verfahrensabschnitte gelangen kann.

Eingedenk der zuvor geschilderten Problematiken befasst sich die vorliegende Erfindung mit der Bereitstellung eines Verfahrens zur Herstellung von Isocyanaten aus den korrespondierenden Aminen, das sich durch eine geringe Neigung zur Bildung von Nebenkomponenten auszeichnet, die zu Ausbeuteverlusten und/oder Qualitätsproblemen und/oder Phosgenentwicklung in eigentlich Phosgen-freien Verfahrensabschnitten führen können.

Gegenstand der Erfindung ist daher ein kontinuierliches Verfahren zur Herstellung eines Isocyanats durch
(i) Umsetzung des korrespondierenden primären Amins mit Phosgen im stöchiometrischen Überschuss in einem Reaktionsraum, wobei die Umsetzung entweder
   in der Flüssigphase in Gegenwart eines inerten Lösungsmittels oder
   in der Gasphase, wobei dem nach Verlassen des Reaktionsraums erhaltenen Verfahrensprodukt ein flüssiges inertes Lösungsmittel enthaltender Strom zugegeben wird,
   durchgeführt wird, sodass ein Rohprodukt **1,** umfassend das gewünschte Isocyanat, inertes Lösungsmittel, Nebenkomponenten mit einem Siedepunkt unter dem des Isocyanats (Leichtsieder), Nebenkomponenten mit einem Siedpunkt über dem des Isocyanats (Hochsieder), Chlorwasserstoff und nicht umgesetztes Phosgen, erhalten wird,
(ii) Auftrennung des Rohprodukts **1** in einen das gewünschte Isocyanat enthaltenden flüssigen Produktstrom **2** und in einen gasförmigen Produktstrom **3,**
(iii) Aufarbeitung des flüssigen Produktstroms **2,** wobei inertes Lösungsmittel, Leichtsieder, Hochsieder, Chlorwasserstoff und Phosgen vom gewünschten Isocyanat abgetrennt werden,
wobei die Abtrennung der Leichtsieder, des Chlorwasserstoffs und des Phosgens in Schritt (iii) innerhalb eines Zeitraums von 30 Sekunden bis 60 Minuten, bevorzugt von 60 Sekunden bis 50 Minuten, besonders bevorzugt von 2 Minuten bis 40 Minuten, nach der Auftrennung des Rohproduktes **1** in Schritt (ii) in die Produktströme **2** und **3** erfolgt und die Temperatur des Produktstroms **2** stets kleiner oder gleich 250 °C, bevorzugt zwischen 100 °C und 250 °C, besonders bevorzugt zwischen 120 °C und 230 °C gehalten wird.

Bevorzugte *primäre Amine* sind solche ausgewählt aus der Gruppe bestehend aus
aliphatischen Aminen (bevorzugt 1,6-Diaminohexan, Methylamin, Ethylamin, Propylamin, Butylamin, Pentylamin und Hexylamin, besonders bevorzugt 1,6-Diaminohexan),
cycloaliphatischen Aminen (bevorzugt Cyclohexylamin, Isophorondiamin, 4,4'-Diaminodicyclohexylmethan, 2,4'-Diaminodicyclohexylmethan, 2,2'-Diaminodicyclohexylmethan und Gemische der Diaminodicyclohexylmethanisomeren, besonders bevorzugt Isophorondiamin, 4,4'-Diaminodicyclohexylmethan, 2,4'-Diaminodicyclohexylmethan, 2,2'-Diaminodicyclohexylmethan und Gemische der Diaminodicyclohexylmethanisomeren),
araliphatischen Aminen (bevorzugt Benzylamin),
und
aromatischen Aminen (bevorzugt Anilin, Chloranilin, Toluylendiamin, 1,5-Diaminonaphthalin, 4,4'-Diaminodiphenylmethan, 2,4'-Diaminodiphenylmethan, 2,2'-Diaminodiphenylmethan, Gemische der Diaminodiphenylmethanisomeren und Gemische der Diaminodiphenylmethanisomeren und deren höheren Homologen [auch allgemein als z. B. MDA, PMDA, Polymer-MDA oder Di- und Polyamine der Diphenylmethanserie bezeichnet, d. h. Gemische von Di- und Polyaminen, wie sie aus der sauer katalysierten Kondensation von Anilin und Formaldehyd erhalten werden], besonders bevorzugt Toluylendiamin).

Ganz besonders bevorzugt wird im erfindungsgemäßen Verfahren Toluylendiamin (TDA) als primäres Amin eingesetzt. TDA wird in der Regel durch Nitrierung von Toluol zu Dinitrotoluol (DNT) und dessen anschließende Hydrierung erhalten. Bevorzugt wird ein Isomerengemisch eingesetzt, das im Wesentlichen aus meta-TDA-Isomeren (m-TDA; d. h. die beiden Aminogruppen stehen zueinander in meta-Position) besteht und von 78 Massen-% bis 82 Massen-% 2,4-TDA und von 18 Massen-% bis 22 Massen-% 2,6-TDA enthält, und das noch weniger als 1 Massen-% des para-TDA-Isomeren (2,5-TDA) enthalten kann. Im erfindungsgemäßen Verfahren zur Umsetzung aromatischer Diamine mit Phosgen ist jedoch ebenfalls der Einsatz von m-TDA-Isomerengemische mit hiervon abweichenden Isomerenverhältnissen wie auch der getrennte Einsatz der technisch reinen 2,4- oder 2,6-TDA-Isomere möglich. Das TDA kann ggf. in geringen Mengen noch Verunreinigungen enthalten.

Unter *Umsetzung im stöchiometrischen Überschuss* wird dabei verstanden, dass mehr als die aufgrund der Stöchiometrie der zugrundeliegenden Reaktionsgleichung berechnete Menge an Phosgen eingesetzt wird. Bevorzugt beträgt der molare Phosgenüberschuss, bezogen auf die vorhandenen primären Aminogruppen, zwischen 1,0 % und 1000 %, besonders bevorzugt zwischen 10 % und 500 % und ganz besonders bevorzugt zwischen 50 % und 350 % der Theorie.

Unter *Reaktionsraum* wird dabei der Raum verstanden, in welchem die Voraussetzungen für eine Reaktion von primärem Amin (bzw. Zwischenprodukten wie z. B. Harnstoffen) mit Phosgen zum gewünschten Isocyanat oder zu einem Gemisch aus dem gewünschten Isocyanat und dem entsprechenden Carbaminsäurechlorid gegeben sind (in Gegenwart von Chlorwasserstoff stehen Isocyanat und Carbaminsäurechlorid grundsätzlich im Gleichgewicht miteinander). Der Reaktionsraum beginnt also an der Stelle, an welcher Amin und Phosgen zum ersten Mal miteinander unter Bedingungen, die eine Reaktion ermöglichen, in Kontakt treten.

Der Reaktionsraum endet im Fall der Flüssigphasenreaktion an der Stelle, an der die Reaktion durch Einleiten geeigneter Maßnahmen (z. B. Temperaturerniedrigung) abgebrochen wird. In der Regel ist der Reaktionsraum durch die räumlichen Abmessungen im Inneren des eingesetzten Apparates vorgegeben.

Der Reaktionsraum endet im Fall der Gasphasenreaktion an der Stelle, an der entweder der aus Produkten, Nebenkomponenten, ggf. nicht umgesetzten Edukten und Zwischenprodukten sowie ggf. zugesetzten inerten Stoffen bestehende Gasstrom in eine Vorrichtung zur Verflüssigung des gebildeten Isocyanats geführt oder die Reaktion durch Einleiten anderer geeigneter Maßnahmen (z. B. Temperaturerniedrigung) abgebrochen wird.

Der Reaktionsraum befindet sich in beiden Verfahrensführungen in einer technischen Vorrichtung zur Durchführung von chemischen Reaktionen, dem *Reaktor.* Es können auch mehrere Reaktoren, parallel oder in Serie geschaltet, verwendet werden. Im einfachsten Fall ist der Reaktionsraum identisch mit dem Innenvolumen des Reaktors bzw. der Reaktoren. Es ist auch vorstellbar, dass ein Reaktor mehrere Reaktionsräume enthält.

Unter *Umsetzung in der Flüssigphase* ist zu verstehen, dass die Amine in der flüssigen Phase zu den Isocyanaten reagieren und im Verlauf der Reaktion sämtliche vorhandenen Komponenten (Edukte, Produkte, Zwischenprodukte, ggf. Nebenkomponenten, ggf. Inertstoffe) während des Durchgangs durch den Reaktionsraum zu mindestens 40,0 Massen-%, bevorzugt zu mindestens 55,0 Massen-%, besonders bevorzugt zu mindestens 65,0 Massen-% und ganz besonders bevorzugt zu mindestens 80,0 Massen-%, jeweils bezogen auf die Gesamtmasse aller im Reaktionsraum vorhandenen Komponenten, in der Flüssigphase verbleiben. Eine ggf. vorhandene Gasphase besteht im Wesentlichen aus HCl und Phosgen.

Unter *Umsetzung in der Gasphase* ist zu verstehen, dass die Amine im gasförmigen Zustand zu den Isocyanaten reagieren und im Verlauf der Reaktion sämtliche vorhandenen Komponenten (Edukte, Produkte, Zwischenprodukte, ggf. Nebenkomponenten, ggf. Inertstoffe) während des Durchgangs durch den Reaktionsraum zu mindestens 95,0 Massen-%, bevorzugt zu mindestens 98,0 Massen-%, besonders bevorzugt zu mindestens 99,0 Massen-% und ganz besonders bevorzugt zu mindestens 99,9 Massen-%, jeweils bezogen auf die Gesamtmasse aller im Reaktionsraum vorhandenen Komponenten, in der Gasphase verbleiben.

*Inerte Lösungsmittel* sind - dies gilt für die Gas- und die Flüssigphasenreaktion - solche, die unter den herrschenden Bedingungen von Temperatur und Druck (siehe weiter unten für Details) mit den vorhandenen Komponenten (Edukte, Produkte, Zwischenprodukte, Nebenkomponenten und/oder Koppelprodukte) nicht in signifikantem Ausmaß, bevorzugt überhaupt nicht, reagieren. Bevorzugt ist das inerte Lösungsmittel ausgewählt aus wenigstens einem Lösungsmittel der Gruppe bestehend aus chlorierten aromatischen Kohlenwasserstoffen (bevorzugt Chlorbenzol, Dichlorbenzol und Trichlorbenzol), aromatischen Kohlenwasserstoffen (bevorzugt Toluol, Xylol und Benzol), Ether (bevorzugt Diphenylether), Sulfoxiden (bevorzugt Dimethylsulfoxid) und Sulfonen (bevorzugt Sulfolan). Chlorierte aromatische Kohlenwasserstoffe sind dabei besonders bevorzugt. Ganz besonders bevorzugt sind ortho-, meta- oder para-Dichlorbenzol sowie Isomerengemische des Dichlorbenzols. Außerordentlich besonders bevorzugt ist ortho-Dichlorbenzol.

Überraschenderweise zeichnet sich das erfindungsgemäße Verfahren durch eine geringe Neigung zur Bildung von Nebenprodukten aus, die zu Ausbeuteverlusten und/oder Qualitätsproblemen und/oder Phosgenentwicklung in eigentlich Phosgen-freien Verfahrensabschnitten führen können.

Durch das erfindungsgemäße Verfahren wird der Anteil chlorhaltiger Spezies im nach der weitestgehenden Entfernung von Leichtsiedern, Chlorwasserstoff und Phosgen erhaltenen Produktstrom zumindest deutlich reduziert. Dies gilt auch für die bereits erwähnten Nebenprodukte mit Chlorformamidin-N-carbonylchlorid-Strukturelementen, die als Phosgen-Carrier in eigentlich Phosgen-freie Abschnitte des Verfahrens gelangen können und bei thermischer Belastung wieder Phosgen abspalten. Da das erfindungsgemäße Verfahren die Bildung der Nebenprodukte mit Chlorformamidin-N-carbonylchlorid-Strukturelementen weitestgehend reduziert, wird vermieden, dass Phosgen in eigentlich Phosgen-freie Verfahrensabschnitte gelangt.

Ausführungsformen der vorliegenden Erfindung werden nachfolgend im Detail beschrieben, wobei die einzelnen Ausführungsformen frei miteinander kombiniert werden können, sofern sich aus dem Kontext nicht eindeutig das Gegenteil ergibt. Weiterhin werden im Folgenden der Flüssigphasen- und der Gasphasenprozess beschrieben. Welche der beiden Verfahrensvarianten zu bevorzugen ist, hängt insbesondere vom einzusetzenden Amin ab. Amine mit einem sehr hohen Siedepunkt, bei deren Verdampfung Zersetzungsreaktionen zu befürchten sind, werden bevorzugt nach dem Flüssigphasenverfahren umgesetzt. Amine, die sich unzersetzt verdampfen lassen, können grundsätzlich nach beiden Verfahren umgesetzt werden. Die Wahl zwischen Flüssig- und Gasphasenverfahren ist in diesen letztgenannten Fällen von verschiedenen, nicht nur technischen, Faktoren abhängig, wie bspw. den wirtschaftlichen Randbedingungen. Das Gasphasenverfahren ist für die Amine ausgewählt aus der Gruppe bestehend aus 1,6-Diaminohexan, Methylamin, Ethylamin, Propylamin, Butylamin, Pentylamin, Hexylamin, Cyclohexylamin, Isophorondiamin, Diaminodicyclohexylmethan (alle Isomere), Gemische der Diaminodicyclohexylmethanisomeren, Benzylamin, Anilin, Chloranilin, Toluylendiamin (alle Isomere), 1,5-Diaminonaphthalin und Diaminodiphenylmethan (alle Isomere) besonders bevorzugt.

Die nachfolgende Beschreibung erfolgt primär anhand des Amins Toluylendiamin. Für den Fachmann ist es ein Leichtes, die nachfolgend genannten Verfahrensdetails ggf. an andere Amine anzupassen.

Die Umsetzung des Toluylendiamins mit Phosgen in **Schritt (i)** kann sowohl nach dem Flüssigphasenals auch nach dem Gasphasenverfahren erfolgen. Das Gasphasenverfahren ist im Fall von Toluylendiamin ganz besonders bevorzugt.

Im Flüssigphasenverfahren wird das Toluylendiamin, ggf. bereits in einem der weiter oben definierten inerten Lösungsmittel gelöst, dem Reaktionsraum mit Temperaturen von -10 °C bis 220 °C, bevorzugt von 0° C bis 200 °C, besonders bevorzugt von 20 °C bis 180 °C, zugeführt. Das Phosgen kann entweder ohne Lösungsmittel oder ebenfalls in einem der weiter oben definierten inerten Lösungsmittel gelöst dem Reaktionsraum mit Temperaturen von -40 °C bis 200 °C, bevorzugt von -30 °C bis 170 °C, besonders bevorzugt von -20 °C bis 150 °C zugeführt werden. Die Vermischung von TDA und Phosgen, ggf. bereits in einem der weiter oben definierten inerten Lösungsmittel gelöst, erfolgt im Flüssigphasenverfahren bevorzugt mittels eines statischen Mischers oder eines dynamischen Mischers. Beispiele für geeignete statische Mischer sind u.a. Düsen bzw. Düsenanordnungen, wie z. B. in DE 17 92 660 A, US 4,289,732 oder US 4,419,295 beschrieben. Beispiele für geeignete dynamische Mischer sind u. a. pumpenähnliche Aggregate, wie beispielsweise Kreiselpumpen (vgl. US 3,713,833) oder spezielle Mischer-Reaktoren (vgl. EP 0 291 819 A, EP 0 291 820 A, EP 0 830 894 A).

Die Umsetzung im Reaktionsraum erfolgt im Flüssigphasenverfahren bei Temperaturen von 0 °C bis 250 °C, bevorzugt von 20 °C bis 200 °C, besonders bevorzugt von 20 °C bis 180 °C, mit mittleren Verweilzeiten des Reaktionsgemisches im Reaktionsraum zwischen 10 s und 5 h, bevorzugt zwischen 30 s und 4 h, besonders bevorzugt zwischen 60 s und 3 h, und bei einem absoluten Druck von max. 100 bar, bevorzugt 1,0 bar bis 70 bar, besonders bevorzugt von 1,0 bar bis 50 bar. Beispiele für erfindungsgemäß einsetzbare Verfahrensführungen im Hinblick auf die Umsetzung im Reaktionsraum sind z. B. in US-A 2007/0299279 (insbesondere S. 7 Abschnitte [0070], [0071], [0089]) und DE-A 103 10 888 (insbesondere S. 5 Abschnitte [0038], [0039]) und darin zitierten Schriften beschrieben.

Im Gasphasenverfahren wird das Toluylendiamin zunächst in die Gasphase überführt. Dies geschieht bevorzugt mit Hilfe eines Verdampfers wie er aus dem Stand der Technik bekannt ist. Das TDA wird auf 200 °C bis 600 °C, bevorzugt 200 °C bis 500 °C, besonders bevorzugt 250 °C bis 450 °C erhitzt, gegebenenfalls mit einem Inertgas wie N₂, He, Ar oder mit den Dämpfen eines der weiter oben definierten inerten Lösungsmittel verdünnt und dem Reaktionsraum zugeführt. Im Gasphasenverfahren wird das Phosgen, ggf. verdünnt mit einem Inertgas wie N₂, He, Ar oder mit den Dämpfen eines der weiter oben definierten inerten Lösungsmittel, dem Reaktionsraum gasförmig mit Temperaturen von 200 °C bis 600 °C, bevorzugt von 200 °C bis 500 °C, besonders bevorzugt von 250 °C bis 450 °C zugeführt. Die Vermischung von TDA und Phosgen erfolgt im Gasphasenverfahren bevorzugt mittels dem Fachmann bekannter statischer oder dynamischer Mischorgane. Bevorzugt ist der Einsatz von Düsen, wie er in EP 1 449 826 B1, insbesondere auf S. 4 Abschnitte [0024], [0025], [0026] uns S. 5 Abschnitt [0027], oder in EP 2 199 277 B1 auf S. 4, Abschnitte [0017], [0018] und S. 5. Abschnitt [0019] beschrieben ist.

Die Umsetzung im Reaktionsraum erfolgt im Gasphasenverfahren bei Temperaturen von 200 °C bis 700 °C, bevorzugt von 200 °C bis 650 °C, besonders bevorzugt von 250 °C bis 600 °C und mit mittleren Verweilzeiten des Reaktionsgemisches im Reaktionsraum zwischen 0,01 s und 120 s, bevorzugt zwischen 0,01 s und 30 s, besonders bevorzugt zwischen 0,05 s und 15 s, und bei einem absoluten Druck von max. 5 bar, bevorzugt 0,5 bar bis 3,0 bar, besonders bevorzugt von 1,0 bar bis 2,0 bar. Nach Verlassen des Reaktionsraumes wird das heiße gasförmige Reaktionsgemisch durch Eindüsen eines oder Durchleiten durch eines der weiter oben definierten inerten Lösungsmittel auf eine Temperatur von 100 °C bis 200 °C, bevorzugt von 150 °C bis 180 °C, abgekühlt und das Isocyanat verflüssigt. Beispiele für erfindungsgemäß einsetzbare Verfahrensführungen sind unter anderem in EP 1 449 826 A1 (insbesondere S. 3 Abschnitte [0012], [0017], [0018], S. 4 Abschnitt [0022] und EP 2 199 277 A1 (insbesondere S. 8 Abschnitte [0054], [0055], [0056], [0057]) beschrieben.

In **Schritt (ii)** wird das in (i) erhaltene Rohprodukt **1,** welches das gewünschte Isocyanat, inertes Lösungsmittel, Leichtsieder, Hochsieder, Chlorwasserstoff und nicht umgesetztes Phosgen enthält, in einen flüssigen Produktstrom **2** und in einen gasförmigen Produktstrom **3,** der im Wesentlichen Chlorwasserstoff und überschüssiges Phosgen enthält, getrennt. Dies geschieht bevorzugt in dem Fachmann bekannten Apparaten und Behältern, die zur Trennung von Gas- und Flüssigphasen geeignet sind. Bevorzugt werden Gas- und Flüssigkeitsabscheider wie z. B. Zyklonabscheider, Umlenkabscheider, Schwerkraftabscheider mit und ohne statischer Abscheidehilfe eingesetzt.

Der in Schritt (ii) erhaltene flüssige Produktstrom **2** enthält neben dem Isocyanat das eingesetzte inerte Lösungsmittel, leichtsiedende Stoffe wie z. B. Phosgen und Chlorwasserstoff, sowie Hoch- und Leichtsieder. Im Allgemeinen enthält der flüssige Produktstrom **2** zwischen 10 Massen-% und 100 Massen-% an Toluylendiisocyanat, zwischen 0 Massen-% und 90 Massen-% an inertem Lösungsmittel, zwischen 0 Massen-% und 5,0 Massen-% an Hochsiedern, zwischen 10 Massen-% und 5,0 Massen-% an Leichtsiedern, zwischen 0 Massen-% und 5,0 Massen-% an Phosgen und zwischen 0 Massen-% und 5,0 Massen-% an gelöstem Chlorwasserstoff, jeweils bezogen auf die Gesamtmasse des flüssigen Produktstromes **2.**

Je nach Ausgestaltung des Schrittes (ii) können auch mehrere Teilströme **2a, 2b,** etc. erhalten werden, bevorzugt werden zwei Teilströme, **2a** und 2b, erhalten. Dies ist dann der Fall, wenn das Rohprodukt **1** zunächst in eine Vorrichtung zur Trennung von Gas- und Flüssigphasen geleitet wird, wobei eine flüssige Phase **2a** und eine Gasphase entsteht, und die Gasphase im Anschluss durch eine Vorrichtung zur Tropfenabscheidung geleitet wird, wobei eine Flüssigphase **2b** und eine von mitgerissenen Tropfen befreite Gasphase **3** entsteht. Der Zusatzstrom **2b** enthält bevorzugt 0 Massen-% bis 5,0 Massen-% Isocyanat, 0 Massen-% bis 10 Massen-% Phosgen und 0 Massen-% bis 100 Massen-% Lösungsmittel. Die Zusammensetzung des Hauptstroms **2a** entspricht bevorzugt der zuvor genannten Zusammensetzung von **2.**

Wird in Schritt (ii) genau ein flüssiger Produktstrom **2** erhalten, so wird dieser in Schritt (iii) zu reinem Isocyanat verarbeitet. Werden zwei oder mehr flüssige Produktströme **2a, 2b,** etc. in Schritt (ii) erhalten, so werden diese entweder zusammengeführt und gemeinsam zu reinem Isocyanat verarbeitet, oder teilweise zusammengeführt und in zwei oder mehr Teilströmen weiter verarbeitet, oder jeder Teilstrom wird separat weiter verarbeitet, wobei mindestens einer der Teilströme zu reinem Isocyanat verarbeitet wird. Dabei ist es auch möglich, dass Teilströme im Zuge ihrer Weiterverarbeitung zu einem späteren Zeitpunkt zusammengeführt werden. Die erfindungsgemäßen Anforderungen an den Zeitraum bis zur Abtrennung der Leichtsieder, des Chlorwasserstoffs und des Phosgens und an die Temperatur in Schritt (iii) gelten für jeden der Produktströme **2a, 2b,** etc. gleichermaßen.

Die Beschreibung des Schrittes (iii) weiter unten erfolgt anhand der Ausführungsform mit genau einem flüssigen Produktstrom **2.** Die Verfahrensdetails gelten jedoch, ggf. mit für den Fachmann selbstverständlichen Anpassungen, für die Verarbeitung mehrerer Teilströme **2a, 2b,** etc. analog.

Im Allgemeinen beträgt die Temperatur des in Schritt (ii) erhaltenen flüssigen Produktstromes **2** maximal 250 °C, bevorzugt maximal 220 °C und insbesondere bevorzugt maximal 200 °C. Weiterhin beträgt die Temperatur von **2** üblicherweise mindestens 60 °C, bevorzugt mindestens 90 °C und insbesondere bevorzugt mindestens 110 °C. Diese Werte gelten für das Flüssig- und das Gasphasenverfahren gleichermaßen.

Des Weiteren wird in Schritt (ii) ein gasförmiger Produktstrom **3** enthaltend im Wesentlichen Phosgen und Chlorwasserstoff erhalten. Die Zusammensetzung dieses Stromes ist abhängig von den genauen Prozessparametern in Schritt (i) und (ii). Detailkenntnisse zur Zusammensetzung von **3** sind für das Verständnis der Erfindung nicht relevant. Der Produktstrom **3** wird im Rahmen der vorliegenden Erfindung wie im Stand der Technik üblich weiter aufgearbeitet. U. a. wird Phosgen abgetrennt und wieder in den Prozess zurückgeführt. Chlorwasserstoff wird ebenfalls abgetrennt und soweit erforderlich gereinigt, um ihn z. B. nach Absorption als wässrige Salzsäure nutzen zu können oder um den Chlorkreislauf zu schließen, indem der Chlorwasserstoff z. B. mittels Elektrolyse oder durch Oxidation in einem Deacon-Prozess wieder in Chlor überführt wird.

Die Aufarbeitung des flüssigen Produktstroms **2** in **Schritt (iii)** erfolgt bevorzugt destillativ. Alternativ können auch andere Verfahren, wie z. B. Kristallisation, Extraktion oder Membranverfahren, für die Aufarbeitung herangezogen werden. Selbstverständlich ist auch eine Kombination verschiedener Verfahren für die Aufarbeitung möglich.

Die bevorzugte destillative Aufarbeitung des flüssigen Produktstroms **2** in Schritt (iii) kann entweder einstufig oder besonders bevorzugt mehrstufig erfolgen. Geeignete Apparate sind z. B. Kolonnen, die ggf. mit geeigneten Einbauten und/oder Füllkörpern, Trennböden und/oder Strukturpackungen versehen sind. Bevorzugt sind Trennwandkolonnen. Geeignete Kombinationen mehrerer Kolonnen bzw. Kolonnentypen sind ebenfalls möglich. Bei Verwendung einer Trennwandkolonne können zwei oder mehr Trennaufgaben bzw. Aufgaben der Aufarbeitung in einem Apparat vereinigt werden. Im Allgemeinen erfolgt die destillative Aufarbeitung bei Sumpftemperaturen im Bereich von 60 °C bis 250°C, bevorzugt 100 °C bis 240°C und besonders bevorzugt von 120 °C bis 230°C. Die absoluten Kopfdrücke liegen im Allgemeinen im Bereich von 1,0 mbar bis 1400 mbar, bevorzugt von 5,0 mbar bis 1013 mbar. Die absoluten Sumpfdrücke liegen höher als die Kopfdrücke und bewegen sich im Bereich von 2,0 mbar bis 2000 mbar, bevorzugt von 7,0 mbar bis 1500 mbar.

Die, bevorzugt destillative, Aufarbeitung in Schritt (iii) umfasst die Gewinnung des Wertproduktes Toluylendiisocyanat (TDI) unter Abtrennung des inerten Lösungsmittels, der Leichtsieder, der Hochsieder, des Phosgens und des Chlorwasserstoffs. Das inerte Lösungsmittel wird bevorzugt soweit erforderlich gereinigt und in den Prozess rezyklisiert. Chlorwasserstoff kann auch chemisch gebunden vorliegen, z. B. als Carbaminsäurechlorid und auch in Gestalt weiterer Chlor-haltiger Spezies. Die Spezies mit chemisch gebundenem Chlorwasserstoff sind im Allgemeinen der Gruppe der Hochsieder zuzurechnen. Carbaminsäurechlorid wird in Schritt (iii) soweit wie möglich unter Gewinnung des Isocyanats gespalten.

Erfindungswesentlich ist, dass in Schritt (iii) die Leichtsieder, der Chlorwasserstoff und das Phosgen innerhalb eines Zeitraums von 30 Sekunden bis 60 Minuten, bevorzugt von 60 Sekunden bis 50 Minuten, besonders bevorzugt von 2 Minuten bis 40 Minuten, nach der Auftrennung des Rohproduktes **1** in Schritt (ii) in die Produktströme **2** und **3** vom gewünschten Isocyanat, in der vorliegenden beispielhaften Beschreibung TDI, abgetrennt werden und die Temperatur des Produktstroms **2** stets kleiner oder gleich 250 °C, bevorzugt zwischen 100 °C und 250 °C, besonders bevorzugt zwischen 120 °C und 230 °C gehalten wird. Die Untergrenze des genannten Zeitraums ist zum einen durch die räumliche Distanz zwischen Reaktion und Aufarbeitung, bevorzugt Destillation gegeben, zum anderen natürlich dadurch, dass eine Mindestverweilzeit in der gewählten Trennvorrichtung, bevorzugt einer Destillationskolonne, nötig ist, um die genannten leichtsiedenden Stoffe abzutrennen.

Dabei gilt die Abtrennung der Leichtsieder, des Chlorwasserstoffs und des Phosgens als im Sinne der Erfindung erfolgt, wenn jeweils mindestens 95,0 Massen-%, bevorzugt mindestens 98,0 Massen-%, besonders bevorzugt mindestens 99,0 Massen-%, ganz besonders bevorzugt mindestens 99,5 Massen-% und außerordentlich besonders bevorzugt 100 Massen-% der Leichtsieder, des Chlorwasserstoffs und des Phosgens abgetrennt sind, jeweils bezogen auf die Masse an Leichtsiedern, die Masse an Chlorwasserstoff und die Masse an Phosgen, die den Reaktionsraum verlässt.

In einer bevorzugten Ausführungsform der Aufarbeitung in Schritt (iii) wird diese mehrstufig durchgeführt, wobei in einer ersten Stufe (iii.a) inertes Lösungsmittel, Leichtsieder, Chlorwasserstoff und Phosgen aus dem Produktstrom 2 durch Destillation abgetrennt werden, so dass ein an inertem Lösungsmittel, Leichtsiedern, Chlorwasserstoff und Phosgen abgereicherter Produktstrom **4** erhalten wird, und in wenigstens einer weiteren Stufe (iii.b) Rein-Isocyanat **5** aus dem Produktstrom **4** destillativ gewonnen wird. Diese Schritte (iii.a) und (iii.b) können in aus dem Stand der Technik an sich bekannten Apparaten durchgeführt werden. Wesentlich ist dabei nur, dass die Abtrennung der Leichtsieder, des Chlorwasserstoffs und des Phosgens in Schritt (iii.a) innerhalb eines Zeitraums von 30 Sekunden bis 60 Minuten, bevorzugt von 60 Sekunden bis 50 Minuten, besonders bevorzugt von 2 Minuten bis 40 Minuten, nach der Auftrennung des Rohproduktes **1** in Schritt (ii) in die Produktströme **2** und **3** erfolgt und die Temperatur des Produktstroms **2** stets kleiner oder gleich 250 °C, bevorzugt zwischen 100 °C und 250 °C, besonders bevorzugt zwischen 120 °C und 230 °C gehalten wird. Die Abmessungen und Anordnung der einzelnen Apparate, die Abmessungen von verbindenden Rohrleitungen und Strömungsgeschwindigkeiten müssen daher so aufeinander abgestimmt werden, dass der erfindungsgemäße Zeitraum bis zur Abtrennung der Leichtsieder, des Chlorwasserstoffs und des Phosgens eingehalten wird. Der Fachmann ist in der Lage, die geeigneten Parameter auf einfache Weise zu ermitteln; ggf. können bei einer gegebenen Produktionsanlage einfache Vorversuche nötig sein. Bevorzugt wird für Schritt (iii.a) eine sog. Entphosgenierkolonne eingesetzt. Bevorzugt wird für Schritt (iii.b) eine dem Fachmann bekannte Destillationskolonne oder Trennwandkolonne zur Feinreinigung eingesetzt. Zur Gewinnung von Isocyanat höchster Reinheit können auch mehrere Destillationskolonnen, wobei verschiedene Kolonnentypen kombiniert werden können, eingesetzt werden. Geeignete Apparate zur Durchführung der Schritte (iii.a) und (iii.b) sind beispielsweise beschrieben in Ullmann's Encyclopedia of Industrial Chemistry (Johann Stichlmair; Distillation, 2. Equipment; publiziert online 2010-04-15, DOI: 10.1002/14356007.o08_o01), EP 1 546 091 A1, US 2,471,134 A, US 2003/0047438 A1, EP 1 371 633 A1, EP 1 371 634 A1, EP 1 413 571 A1, EP 2 210 873 A1, WO 2010/039972 A2, DE 19 23 214 A1, EP 1 475 367 B1. Wie solche Apparate grundsätzlich zu betreiben sind, ist dem Fachmann bekannt.

In der bevorzugten mehrstufigen Aufarbeitung in den Schritten (iii.a) und (iii.b) entspricht die erfindungsgemäß einzuhaltende Zeitspanne zwischen der Auftrennung des Rohprodukts **1** in den flüssigen Produktstrom **2** und den gasförmigen Produktstrom **3** der mittleren Verweilzeit von in Schritt (i) gebildetem Isocyanat zwischen dem Austrag von Strom **2** aus einer Vorrichtung zur Trennung von Flüssig- und Gasphasen (*Schritt (ii)*) und dem Austrag des an inertem Lösungsmittel, Leichtsiedern, Chlorwasserstoff und Phosgen abgereicherten Produktstroms **4** aus der Entphosgenierkolonne (*Schritt (iii.a)*)*.* Die Vorrichtung zur Trennung von Flüssig- und Gasphasen kann dabei in den Reaktor von Schritt (i) integriert sein, sodass die erfindungsgemäße Zeitspanne zwischen der Auftrennung des Rohprodukts **1** in den flüssigen Produktstrom **2** und den gasförmigen Produktstrom **3** der mittleren Verweilzeit von in Schritt (i) gebildetem Isocyanat zwischen dem Austrag von Strom **2** aus dem Reaktor und dem Austrag des an inertem Lösungsmittel, Leichtsiedern, Chlorwasserstoff und Phosgen abgereicherten Produktstroms **4** aus der Entphosgenierkolonne entspricht. In dieser Variante betrifft die Erfindung demnach ein Verfahren, bei dem Schritt (iii.a) in einer Entphosgenierkolonne durchgeführt wird und Schritt (iii.b) in einer Kolonne zur Feinreinigung durchgeführt wird, und bei dem die Verweilzeit von in Schritt (i) gebildetem Isocyanat nach der Auftrennung des Rohproduktes **1** in Schritt (ii) bis zum Austrag des an inertem Lösungsmittel, Leichtsiedern, Chlorwasserstoff und Phosgen abgereicherten Produktstroms **4** aus der Entphosgenierkolonne in Schritt (iii.a) 30 Sekunden bis 60 Minuten, bevorzugt von 60 Sekunden bis 50 Minuten, besonders bevorzugt von 2 Minuten bis 40 Minuten, beträgt.

Auf diese Weise wird ein flüssiger, an inertem Lösungsmittel, Leichtsiedern, Chlorwasserstoff und Phosgen abgereicherter Produktstrom **4** erhalten. Bevorzugt enthält Strom **4,** jeweils bezogen auf die Gesamtmasse dieses Stromes,
40 Massen-% bis 100 Massen-%, besonders bevorzugt 60 Massen-% bis 100 Massen-%, ganz besonders bevorzugt 70 Massen-% bis 100 Massen-% des gewünschten Isocyanats, in der vorliegenden beispielhaften Beschreibung TDI,
0,10 Massen-% bis 10 Massen-%, bevorzugt 0,50 Massen-% bis 7,0 Massen-%, besonders bevorzugt 1,0 Massen-% bis 5,0 Massen-% Hochsieder,
0 Massen-% bis 60 Massen-%, besonders bevorzugt 0 Massen-% bis 40 Massen-%, ganz besonders bevorzugt 0 Massen-% bis 30 Massen-% Lösungsmittel,
0 Massen-ppm bis 1500 Massen-ppm (0-2%), bevorzugt 0 Massen-ppm bis 1200 Massen-ppm (0-1,2%), besonders bevorzugt 0 Massen-ppm bis 1000 Massen-ppm (0-1%) Leichtsieder,

0 Massen-ppm bis 1000 Massen-ppm, bevorzugt 0 Massen-ppm bis 800 Massen-ppm, besonders bevorzugt 0 Massen-ppm bis 600 Massen-ppm Phosgen,
0 Massen-ppm bis 1000 Massen-ppm, bevorzugt 0 Massen-ppm bis 800 Massen-ppm, besonders bevorzugt 0 Massen-ppm bis 600 Massen-ppm Chlorwasserstoff.

Dieser Strom **4** wird anschließend destillativ weiter aufgearbeitet, um Rein-Isocyanat **5,** in der vorliegenden beispielhaften Beschreibung Rein-TDI, zu erhalten. Diese Reindestillation kann nach allen aus dem Stand der Technik bekannten Arten erfolgen. Beispiele werden z. B. in EP 1 371 634 A1 oder den dort zitierten Literaturstellen bzw. Anmeldungen beschrieben.

In den vorstehend beispielhaft beschriebenen Ausführungsformen wird Toluylendiisocyanat (TDI) als Produkt erhalten, wobei die Isomerenverteilung im Wesentlichen der des eingesetzten Toluylendiamins entspricht.

Werden andere Amine zu den entsprechenden Isocyanaten umgesetzt, so sind ggf. Abwandlungen von den zuvor im Einzelnen geschilderten Verfahrensdetails vorzunehmen, was jedoch für den Fachmann eine Routineoperation ist.

Durch die erfindungsgemäße Begrenzung der Temperatur und des Zeitraums zwischen der Trennung des Rohprodukts **1** in die Produktströme **2** und **3** in Schritt (ii) und der Abtrennung der Leichtsieder, des Chlorwasserstoffs und des Phosgens vom gewünschten Isocyanat in Schritt (iii) wird die thermisch induzierte Bildung von Carbodiimide einerseits und die Bildung von Nebenkomponenten mit Chlorformamidin-N-carbonylchlorid-Strukturelementen andererseits weitestgehend vermieden. Solche Nebenkomponenten könnten ansonsten zu Ausbeuteverlusten und/oder Qualitätsproblemen und/oder Phosgenentwicklung in eigentlich Phosgen-freien Verfahrensabschnitten führen. Das erfindungsgemäße Verfahren reduziert also Ausbeuteverluste und damit wirtschaftliche Nachteile auf ein Minimum.

### Beispiele

Nachfolgende Beispiele zeigen die Wichtigkeit der Abtrennung von Leichtsiedern, Chlorwasserstoff und Phosgen innerhalb der erfindungsgemäßen Zeitspanne. Zur Demonstration der Bedeutung dieses Parameters wurde in allen Beispielen der Gehalt an *chemisch gebundenem* Phosgen in Isocyanat-Proben ermittelt. "Chemisch gebunden" liegt Phosgen bspw. in Carbodiimiden vor. Je größer der Gehalt an chemisch gebundenem Phosgen in einem rohen Isocyanat-Strom ist, desto unselektiver ist die Umsetzung des Amins verlaufen und desto größer ist die Gefahr, dass Phosgen in an sich Phosgen-freie Verfahrensabschnitte "verschleppt" wird.

In allen Beispielen wurde jeweils ein Gemisch aus 80 % 2,4-TDA und 20 % 2,6-TDA in einem Gasphasenverfahren wie in EP 0 570 799 B1 beschrieben phosgeniert *(Schritt (i)).*

Das Rohprodukt **1** wurde in einer Reaktionsabbruchszone ("Quenche") durch Eindüsen von ortho-Dichlorbenzol (ODB) als inertem Lösungsmittel in einen flüssigen Produktstrom **2** und einen gasförmigen Produktstrom **3** getrennt *(Schritt (ii)).*

Der so erhaltene flüssige Reaktoraustrag **2** enthielt **87 Massen-ppm (0,0087 Massen-%)** chemisch gebundenes Phosgen. Zur Bestimmung des Gehalts an chemisch gebundenem Phosgen wurde in allen Beispielen wie folgt vorgegangen:
Alle Proben wurden zunächst mittels Durchleitung von trockenem Stickstoff (40 1/h) für 8 Stunden bei max. 30 °C von evtl. noch in Restmengen vorhandenem *gelöstem* Phosgen befreit. Danach wurden jeweils 200 g der so vorbereiteten Proben in einem Rundkolben mit Gaseinleitungsrohr und Rückflusskühler unter Rühren innerhalb von 30 min auf 180 °C erhitzt und für weitere 90 min unter Rühren bei dieser Temperatur gehalten. Während des gesamten Versuchslaufs wurde ein Stickstoffstrom von 10 1/h durch die Rohproduktlösung geleitet, um chemisch gebundenes und infolge der thermischen Belastung wieder freigesetztes Phosgen aus der Lösung auszutreiben und in eine Kaskade von Methanol in definierter Menge enthaltenden Waschflaschen zu überführen. Phosgen reagiert mit dem Methanol zu Dimethylcarbonat, das mittels Gaschromatographie (GC) quantitativ analysiert werden kann. Zur genauen Quantifizierung wurde das Methanol mit Benzophenon als internem Standard für die GC-Analytik versetzt.

### Beispiel 1 (erfindungsgemäß):

Eine Probe des flüssigen Reaktoraustrags **2** wurde kontinuierlich in eine Entphosgenierkolonne überführt und von inertem Lösungsmittel, Leichtsiedern, Chlorwasserstoff und Phosgen befreit *(Schritt (iii*.*a))*, wobei ein vorgereinigter Isocyanat-Strom **4** als Sumpfprodukt der Entphosgenierkolonne erhalten wurde.

| | |
|---|---|
| Temperatur des Stroms **2** bei Eintritt in die Entphosgenierkolonne: | 163 °C. |
| Temperatur im Sumpf der Entphosgenierkolonne: | 180 °C. |
| Absoluter Druck am Kopf der Entphosgenierkolonne: | 680 mbar |

Die mittlere Verweilzeit zwischen Austrag von Strom **2** aus dem Reaktor und Austrag von Strom **4** aus dem Sumpf der Entphosgenierkolonne betrug ca. 20 min. Bezogen auf TDI enthielt Strom **4 58 Massen-ppm (0,0058 Massen-%)** chemisch gebundenes Phosgen. Dieses Beispiel zeigt, dass bei einer erfindungsgemäßen Verweilzeit zwischen dem Erhalt des flüssigen Produktstroms **2** und der Abtrennung der Leichtsieder, des Phosgens und des Chlorwasserstoffs der Gehalt an chemisch gebundenem Phosgen praktisch unverändert ist (dass der Wert nominell sogar geringer wurde, ist auf Messschwankungen zurückzuführen).

### Beispiel 2 (Vergleichsheispiel):

Eine weitere Probe des flüssigen Reaktoraustrags **2** wurde unter kontinuierlicher Zuführung von Phosgen für 3 h bei 180°C und Normaldruck behandelt, um die Bedingungen einer nicht erfindungsgemäßen Verweilzeit zwischen Erhalt des Stroms **2** und Abtrennung der Leichtsieder, des Phosgens und des Chlorwasserstoffs möglichst realitätsnah nachzustellen.

Bezogen auf TDI enthielt die Probe **485 Massen-ppm (0,0485 Massen-%)** chemisch gebundenes Phosgen.

Die Beispiele zeigen, dass sich der Gehalt an chemisch gebundenem Phosgen bei erfindungsgemäßer Vorgehensweise nicht nachteilig verändert, während bei einer zu großen Verweilzeit zwischen Erhalt des Stroms **2** und Abtrennung der Leichtsieder, des Phosgens und des Chlorwasserstoffs der Gehalt an chemisch gebundenem Phosgen signifikant ansteigt.

## Patentansprüche

1. Kontinuierliches Verfahren zur Herstellung eines Isocyanats durch
(i) Umsetzung des korrespondierenden primären Amins mit Phosgen im stöchiometrischen Überschuss in einem Reaktionsraum, wobei die Umsetzung entweder
in der Flüssigphase in Gegenwart eines inerten Lösungsmittels oder
in der Gasphase, wobei dem nach Verlassen des Reaktionsraums erhaltenen Verfahrensprodukt ein flüssiges inertes Lösungsmittel enthaltender Strom zugegeben wird,
durchgeführt wird, sodass ein Rohprodukt **1,** umfassend das gewünschte Isocyanat, inertes Lösungsmittel, Nebenkomponenten mit einem Siedepunkt unter dem des Isocyanats (Leichtsieder), Nebenkomponenten mit einem Siedpunkt über dem des Isocyanats (Hochsieder), Chlorwasserstoff und nicht umgesetztes Phosgen, erhalten wird,
(ii) Auftrennung des Rohprodukts **1** in einen das gewünschte Isocyanat enthaltenden flüssigen Produktstrom **2** und in einen gasförmigen Produktstrom **3,**
(iii) Aufarbeitung des flüssigen Produktstroms **2,** wobei inertes Lösungsmittel, Leichtsieder, Hochsieder, Chlorwasserstoff und Phosgen vom gewünschten Isocyanat abgetrennt werden,
**dadurch gekennzeichnet, dass**
die Abtrennung der Leichtsieder, des Chlorwasserstoffs und des Phosgens in Schritt (iii) innerhalb eines Zeitraums von 30 Sekunden bis 60 Minuten nach der Auftrennung des Rohproduktes **1** in Schritt (ii) in die Produktströme **2** und **3** erfolgt und die Temperatur des Produktstroms **2** stets kleiner oder gleich 250 °C gehalten wird.

2. Verfahren nach Anspruch 1, bei dem die Aufarbeitung in Schritt (iii) mehrstufig durchgeführt wird, wobei in einer ersten Stufe (iii.a) inertes Lösungsmittel, Leichtsieder, Chlorwasserstoff und Phosgen aus dem Produktstrom 2 durch Destillation abgetrennt werden, sodass ein an inertem Lösungsmittel, Leichtsiedern, Chlorwasserstoff und Phosgen abgereicherter Produktstrom **4** erhalten wird, und in wenigstens einer weiteren Stufe (iii.b) Rein-Isocyanat **5** aus dem Produktstrom **4** destillativ gewonnen wird.

3. Verfahren nach Anspruch 2, bei dem Schritt (iii.a) in einer Entphosgenierkolonne durchgeführt wird und Schritt (iii.b) in einer Kolonne zur Feinreinigung durchgeführt wird, und bei dem die Verweilzeit von in Schritt (i) gebildetem Isocyanat nach der Auftrennung des Rohproduktes **1** in Schritt (ii) bis zum Austrag des an inertem Lösungsmittel, Leichtsiedern, Chlorwasserstoff und Phosgen abgereicherten Produktstroms **4** aus der Entphosgenierkolonne in Schritt (iii.a) 30 Sekunden bis 60 Minuten beträgt.

4. Verfahren nach einem der Ansprüche 1 bis 3, bei dem die Umsetzung des primären Amins mit Phosgen im Reaktionsraum in Schritt (i) in der Gasphase durchgeführt wird.

5. Verfahren nach einem der Ansprüche 1 bis 4, bei dem als primäres Amin Toluylendiamin eingesetzt wird.

## Claims

1. Continuous method for preparing an isocyanate by
(i) reacting the corresponding primary amine with phosgene in stoichiometric excess in a reaction chamber, wherein the reaction is carried out either
in the liquid phase in the presence of an inert solvent or
in the gas phase, wherein a stream comprising a liquid inert solvent is added to the process product after leaving the reaction chamber,
such that a crude product **1** is obtained comprising the desired isocyanate, inert solvent, secondary components having a boiling point below that of the isocyanate (low boilers), secondary components having a boiling point above that of the isocyanate (high boilers), hydrogen chloride and unreacted phosgene,
(ii) separating the crude product **1** into a liquid product stream **2** containing the desired isocyanate and into a gaseous product stream **3,**
(iii) working-up the liquid product stream **2,** wherein inert solvent, low boilers, high boilers, hydrogen chloride and phosgene are removed from the desired isocyanate,
**characterized in that**
the low boilers, the hydrogen chloride and the phosgene are removed in step (iii) within a period of 30 seconds to 60 minutes following the separation of the crude product **1** in step (ii) into the product streams **2** and **3,** and the temperature of the product stream **2** is always maintained below or equal to 250°C.

2. Method acording to Claim 1, in which the workup in step (iii) is carried out in more than one stage, wherein inert solvent, low boilers, hydrogen chloride and phosgene are removed from the product stream **2** by distillation in a first stage (iii.a), such that a product stream **4** depleted in inert solvent, low boilers, hydrogen chloride and phosgene is obtained, and in at least one further stage (iii.b) pure isocyanate **5** is obtained from the product stream **4** by distillation.

3. Method acording to Claim 2, in which step (iii.a) is carried out in a dephosgenation column and step (iii.b) is carried out in a column for final purification, and in which the residence time of isocyanate formed in step (i) after the separation of the crude product **1** in step (ii) up to the effluent of the product stream **4** depeleted in inert solvent, low boilers, hydrogen chloride and phosgene from the dephosgenation column in step (iii.a) is 30 seconds to 60 minutes.

4. Method acording to any of Claims 1 to 3, in which the reaction of the primary amine with phosgene in the reaction chamber in step (i) is carried out in the gas phase.

5. Method acording to any of Claims 1 to 4, in which the primary amine used is toluylenediamine.

## Revendications

1. Procédé continu de fabrication d'un isocyanate par
(i) la mise en réaction de l'amine primaire correspondante avec du phosgène en excès stoechiométrique dans une chambre de réaction, la réaction étant réalisée soit
dans la phase liquide en présence d'un solvant inerte, soit
dans la phase gazeuse, un courant contenant un solvant inerte liquide étant ajouté au produit de procédé obtenu à la sortie de la chambre de réaction,
de manière à obtenir un produit brut 1, comprenant l'isocyanate souhaité, un solvant inerte, des composants secondaires ayant un point d'ébullition inférieur à celui de l'isocyanate (composants de point d'ébullition bas), des composants secondaires ayant un point d'ébullition supérieur à celui de l'isocyanate (composants de point d'ébullition élevé), du chlorure d'hydrogène et du phosgène non réagi,
(ii) la séparation du produit brut 1 en un courant de produits liquide contenant l'isocyanate souhaité 2 et en un courant de produits gazeux 3,
(iii) le traitement du courant de produits liquide 2, le solvant inerte, les composants de point d'ébullition bas, les composants de point d'ébullition élevé, le chlorure d'hydrogène et le phosgène étant séparés de l'isocyanate souhaité,
**caractérisé en ce que**
la séparation des composants de point d'ébullition bas, du chlorure d'hydrogène et du phosgène à l'étape (iii) a lieu en une durée de 30 secondes à 60 minutes après la séparation du produit brut 1 à l'étape (ii) en les courants de produits 2 et 3, et la température du courant de produits 2 est toujours maintenue inférieure ou égale à 250 °C.

2. Procédé selon la revendication 1, selon lequel le traitement à l'étape (iii) est réalisé en plusieurs étapes, lors d'une première étape (iii.a), le solvant inerte, les composants de point d'ébullition bas, le chlorure d'hydrogène et le phosgène étant séparés du courant de produits 2 par distillation, de manière à obtenir un courant de produits 4 appauvri en solvant inerte, composants de point d'ébullition bas, chlorure d'hydrogène et phosgène, et lors d'au moins une étape supplémentaire (iii.b), de l'isocyanate pur 5 étant obtenu par distillation à partir du courant de produits 4.

3. Procédé selon la revendication 2, selon lequel l'étape (iii.a) est réalisée dans une colonne de déphosgénation et l'étape (iii.b) dans une colonne pour la purification fine, et selon lequel le temps de séjour de l'isocyanate formé à l'étape (i) après la séparation du produit brut 1 à l'étape (ii) jusqu'au déchargement du courant de produits 4 appauvri en solvant inerte, composants de point d'ébullition bas, chlorure d'hydrogène et phosgène de la colonne de déphosgénation à l'étape (iii.a) est de 30 secondes à 60 minutes.

4. Procédé selon l'une quelconque des revendications 1 à 3, selon lequel la réaction de l'amine primaire avec du phosgène dans la chambre de réaction à l'étape (i) est réalisée dans la phase gazeuse.

5. Procédé selon l'une quelconque des revendications 1 à 4, selon lequel de la toluylène-diamine est utilisée en tant qu'amine primaire.
